Europäisches Patentamt

European Patent Office

Office européen des brevets

(19)

(11) Publication number: **0 091 522**

**A2**

(12)

# EUROPEAN PATENT APPLICATION

(21) Application number: 82303324.6

(22) Date of filing: 25.06.82

(51) Int. Cl.³: **A 61 B 5/08**

(30) Priority: 14.04.82 US 368238

(43) Date of publication of application:
19.10.83 Bulletin 83/42

(84) Designated Contracting States:
DE FR GB SE

(71) Applicant: THE HOSPITAL FOR SICK CHILDREN
555 University Avenue
Toronto Ontario M5G 1X8(CA)

(72) Inventor: Volgyesi, George Andrew
36 Gatehead Road
Willowdale Ontario, M2J 2P5(CA)

(74) Representative: Feakins, Graham Allan et al,
Haseltine Lake & Co. Hazlitt House 28, Southampton
Buildings Chancery Lane
London WC2A 1AT(GB)

(54) Respiration monitor.

(57) This invention relates to a respiration monitor primarily intended to be used in hospitals to provide an alarm signal in the event of cessation of breathing by a patient. The monitor includes a sensor (10,10') having a housing (12,12') which defines an air passageway (14,14') and which is adapted to be placed in communication with the airway of a patient so that air inhaled and exhaled by the patient will flow through said passageway (14,14'). A pair of electrical conductors (26,28) are located in the passageway in the path of air flowing therethrough in use. The conductors are insulated from one another and form a capacitor having a dielectric partially formed by the air between said conductors (26,28), so that changes in the humidity of the air in said passageway (14,14') during inhalation and exhalation by the patient in use will cause corresponding cyclic variations in the capacitance of said capacitor. The monitor also includes electrical circuit means coupled to the conductors and adapted to provide an alarm signal in the event of cessation of said cyclic variations in capacitance.

FIG. 1

would not be suitable for use as a respiration monitor for a number of reasons. A principal disadvantage of this form of device is that the device would be sensitive only at the intersections of the wires. In practice, moisture from a patient's breath would tend to collect at these sensitive areas and it would be held at those areas by surface tension. This accumulated water would rapidly cause failure of the device due to saturation. Also, when wet, motion of the sensor or gusts or air moving droplets of water would probably result in spurious capacitive signals which could lead to failure to alarm. It goes without saying that such failure could have extremely severe consequences in a respiration monitor and for these reasons it is believed that use of this form of device would be dangerous.

An object of the present invention is to provide an improved respiration monitor.

The monitor provided by the invention includes a sensor having a housing which defines an air passageway and which is adapted to be placed in communication with the airway of a patient so that air inhaled and exhaled by the patient will flow through said passageway, and a sensor element located in the passageway in the path of air flowing therethrough in use. The sensor element comprises a pair of insulated wires twisted closely together in at least substantially continuous line contact with one another and forming a capacitor having a dielectric partially formed by the air between said conductors, so that changes in the humidity of the air in said passageway

3

during inhalation and exhalation by the patient in use will cause corresponding cyclic variations in the capacitance of said capacitor. The monitor also includes electrical circuit means coupled to the conductors and adapted to provide an alarm signal in the event of cessation of said cyclic variations in capacitance.

In order that the invention may be more clearly understood, reference will now be made to the accompanying drawings which illustrate preferred embodiments of the invention by way of example, and in which:

Fig. 1 is a vertical sectional view through the sensor of a respiration monitor according to a first embodiment of the invention;

Fig. 2 is a perspective view of part of the sensor element of the sensor shown in Fig. 1;

Fig. 3 is a diagram of an electrical circuit incorporating the sensor shown in the previous views;

Fig. 4 is a view similar to Fig. 1 showing a sensor in accordance with a further embodiment of the invention;

Fig. 4a is a perspective view of part of Fig. 4; and

Fig. 5 is a diagram of a preferred form of electrical circuit incorporating the sensor if Fig. 4.

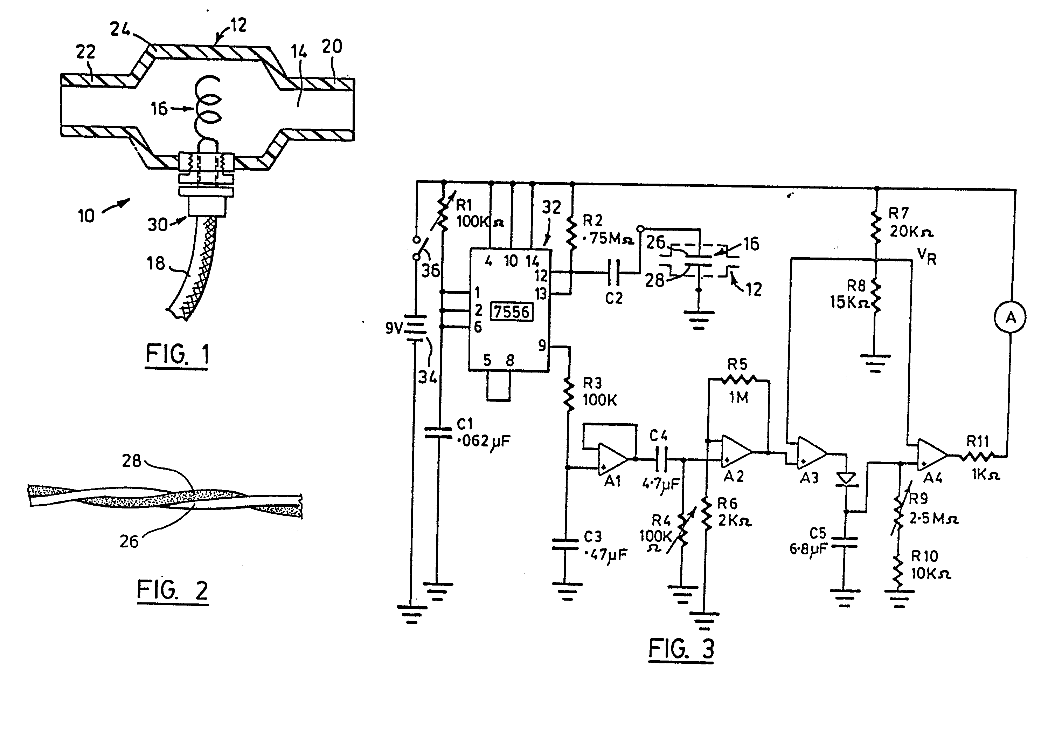

Referring first to Fig. 1, the sensor is generally indicated at 10 and includes a housing 12 which defines an air passageway 14, and a sensor element 16 which is disposed in passageway 14 and which comprises a pair of electrical conductors forming a capacitor as will be more particularly described. The sensor element 16 is connected by a coaxial cable 18 to the electrical circuit shown in Fig. 3. In that view, the sensor housing is indicated in dotted outline at 12 and the sensor element is shown in the form of a capacitor denoted 16.

Referring back to Fig. 1, the sensor housing is designed to be placed in communication with the airway of a patient so that air inhaled and exhaled by the patient will flow through passageway 14. In this particular embodiment, housing 12 is in the form of a plastic moulding and has tubular end portions 20 and 22 and an enlarged intermediate portion 24 which houses the sensor element. Passageway 14 opens at both ends through respective tubular end portions 20 and 22 of housing 12. The housing can be connected with the airway of a patient in various ways. For example, housing end portions 20 and 22 may receive breathing tubes which can be fitted over or into the said portions and used to connect the sensor with, say, a face mask, ventilator or other external piece of equipment. In some cases, it may be desirable to perforate housing 12 to allow entry of ambient air for dissipating condensed moisture from the sensor element 16. In other cases, this effect can be achieved by leaving one end portion of the housing open.

Sensor element 16 is formed by two conductors in the form of insulated wires which are twisted together as shown in Fig. 2 and then formed into the spiral shape shown in Fig. 1. The conductors are denoted 26 and 28 respectively in each of Figs. 2 and 3. It will be seen from Fig. 1 that the wires are mounted in a connector 30 fitted into the enlarged centre portion 24 of housing 12. Cable 18 is connected to the conductors 26 and 28 at connector 30 and the connector maintains the conductors insulated from one another. A coaxial cable is employed to screen out extraneous electrical fields which might affect the capacitance of the sensor element.

Sensor element 16 is positioned in passageway 14 in the path of air which will flow through the passageway as the patient inhales and exhales. In an alternative embodiment, the sensor element could be arranged so that its spiral shape is coaxial with passageway 14 so as to minimize any possible obstruction of the patient's airway, although in practice, the arrangement shown has been found to be entirely satisfactory. In any event, it will be appreciated that the twisted wires in effect form a capacitor having a dielectric partially formed by the air between the conductors of the two wires (the insulation on the wires simply prevents shorting). The monitor operates on the principle that sensor element 16 will respond to changes in the humidity of the air flowing through passageway 14 during the inhalation/exhalation cycle of the patient. During inhalation, the air flowing along passageway 14 will be essentially dry ambient air, while during exhalation the air will be relatively humid, having collected water vapour from the patient. It is believed that, during exhalation, water vapour from the air will condense on the conductors of sensor element 16, changing the dielectric constant of the capacitor formed by the two wires. During inhalation, that condensed water vapour will be removed from the wires by the essentially dry air drawn in by the patient. The dielectric constant of dry air is 1 while that of water is approximately 80. Accordingly, significant changes in capacitance will occur as between the inhalation and exhalation portions of the breathing cycle.

The electrical circuit shown in Fig. 3 is coupled to the conductors 26 and 28 and is adapted to provide an alarm signal in the event of cessation of these cyclic variations in capacitance. The circuit includes a commercially available chip indicated by reference numeral 82 and designated as a 7556 dual timing circuit available from ICM (Intersil). Of course, equivalent chips from other sources could alternatively be used. The circuit also includes four differential amplifiers denoted $A_1$, $A_2$, $A_3$ and $A_4$. These components can also be obtained in the form of a single chip known as a 324 QUAD OP-AMP. The circuit also includes an alarm denoted A responsive to a predetermined low level signal from amplifier $A_4$. Alarm A is of the type which is sold under the trade mark SONALERT. Various resistors and capacitors are also included in the circuit and have the assigned values indicated in Fig. 3. Power for the circuit is provided by a nine volt battery indicated at 34 and a main ON/OFF switch 36 is also provided.

One half of the dual timing circuit 32 has inputs controlled by a capacitor $C_1$ and resistor $R_1$. The circuit provides a regular, repeated "clock" pulse. The cyclic capacitance variations of sensor 10 pulse-width modulate the output of the second half of the timing circuit at terminal 9. These width modulated pulses are detected and buffered by resistor $R_3$, capacitor $C_3$, and amplifier $A_1$ resulting in a corresponding amplitude modulated signal from amplifier $A_1$. The A.C. component of this signal is delivered to amplifier $A_2$ and amplified. The gain provided by amplifier $A_2$ is adjustable by means of a potentiometer $R_4$.

The amplified signal from $A_2$ is fed to amplifier $A_3$; that amplifier will accordingly receive an input signal at each expiration. The input signal is compared with a reference voltage $V_R$ and will normally exceed the reference voltage during each expiration so that the amplifier will be repeatedly triggered while the patient is breathing. The output pulses from amplifier $A_3$ are fed to the fourth amplifier $A_4$ which compares them with reference voltage $V_R$ whose output is high while the patient is breathing normally. Since both inputs to the alarm A are high the alarm remains silent. At the same time, the output pulses from amplifier $A_3$ repeatedly re-

charge capacitor $C_5$. That capacitor is continuously discharging through resistors $R_9$ and $R_{10}$, but while the patient keeps breathing, the recharging pulses will prevent capacitor $C_5$ discharging to a level below the reference $V_R$. If breathing should stop, capacitor $C_5$ will discharge to a level below $V_R$ causing the output of amplifier $A_4$ to go low, activating the alarm A. Resistor $R_9$ is adjustable so that the time which elapses between the last expiration of the patient and sounding of the alarm can be adjusted appropriately.

While other forms of electrical circuit may be employed in place of the circuit shown in Fig. 3, that particular circuit has the advantage of relatively simplicity and ease of manufacture from commercially available components. At the same time, by virtue of the digital nature of the circuit, a counter can be added to the circuit relatively easily to provide an indication of respiration rate if required.

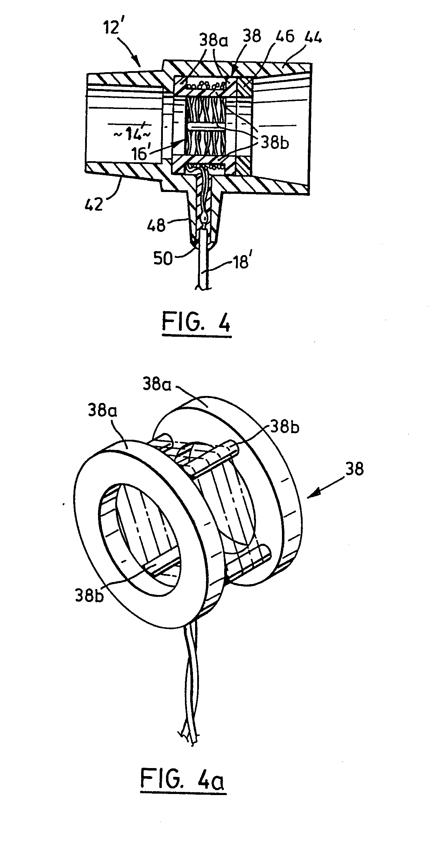

Fig. 4 illustrates a sensor according to a further embodiment of the invention and will now be described. Primed reference numerals have been used in Fig. 4 to denote parts which correspond with Fig. 1.

The sensor as shown in Fig. 4 is generally denoted 10' and includes a housing 12' which defines an air passageway 14', and a sensor element 16'. The sensor element is formed by two insulated conductors which are twisted together in the manner shown in Fig. 2 and then formed into the general shape of a spiral helix of constant diameter. The sensor element is then disposed in air passageway 14' with its axis generally coaxial with the air passageway. It has been found that such an arrangement may be preferred from a practial viewpoint. Not only is any possible obstruction of the patient's airway minimized as discussed above, but the sensor element is firmly retained in its housing. At least from a psychological viewpoint, the sensor element of Fig. 1 has the disadvantage that it may be perceived to be somewhat fragile and susceptible to dislodgement into the patient's airway, although it should be emphasized that such dislodgement has never occurred in practice and it is believed extremely unlikely that it could occur.

Referring back to Fig. 4, the two conductors which form sensor element 16 are in fact spirally wound around a bobbin which is denoted 38 in Fig. 4 and which is shown in perspective in Fig. 4a. For clarity of illustration, the size of the conductors as shwon in Fig. 4 is greatly exaggerated. The sensor element 13 indicated partly in ghost outline in Fig. 4a. Bobbin 38 comprises a pair of ring shaped members 38a joined by a number equally spaced arms 38b. The arms are externally grooved to locate the sensor element. If necessary, a suitable adhesive can be used to secure the element to the bobbin. Alternately, the sensor element could pass through small holes in the arms.

Sensor housing 12' is of tubular form and comprises two coaxial generally cylindrical sections which meet at a shoulder denoted 40. The two cylindrical sections denoted 42 and 44 respectively have complementary male and female shapes. Preferably, the sections 42 and 44 are shaped as standard 15 millimeter gas tubing connectors used in hospitals.

Bobbin 38, carrying the sensor element 16' is inserted into the housing 12' through the larger cylindrical portion 44 and seated against shoulder 40 in manufacture. A retaining ring 46 is then inserted against bobbin 38 and may be held in place by a suitable adhesive.

A coaxial cable 18' is lead into housing portion 44 through a sleeve 48 which is moulded integrally with housing 12' and to which cable 18' is secured by a suitable adhesive as indicated at 50. The conductors of the sensor element are connected one to the screen and the other to the conductor of cable 18'. In fact, each sensor conductor is connected at both ends to cable 18' so that each conductor in effect forms an insulated loop. The connections between the sensor and cable 18' are located in sleeve 48 and are covered by the adhesive 50 (which insulates the connections).

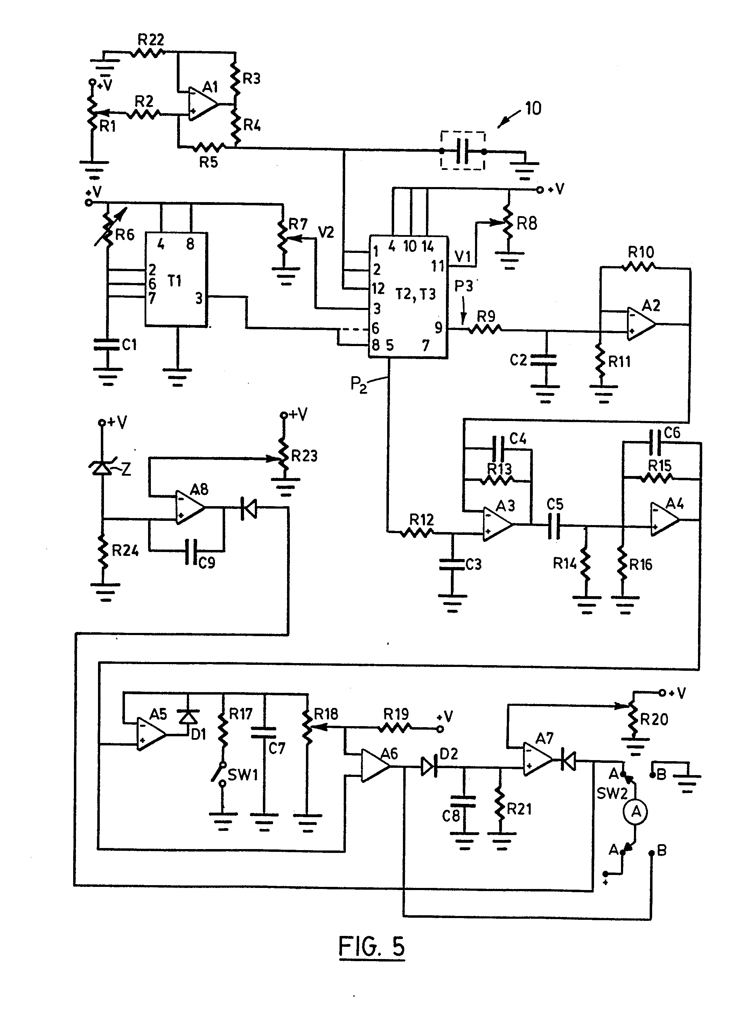

Reference will now be made to Fig. 5 in describing an alternative form of electrical circuit which may be used to provide an alarm signal in the event of cessation of breathing monitored by a sensor of the form provided by the invention. The circuit of Fig. 5 incorporates a number of practical improvements as compared with the circuit of Fig. 3. A principal one of these improvements is that the circuit is designed to minimize the effects of motion of

11

drops of water on the sensor element or other sources of stray capacitance signals which might tend to cause failure to alarm during apnea. The circuit also incorporates modifications designed to permit an audible signal to be given at each expiration e.g. for testing purposes, and also to give an alarm signal indicating low battery voltage.

The virtual elimination of the effect of stray capacitance variations is based on the experimental observation that the dielectric properties of a thin layer of water such as the fine mist deposited on the insulation of the sensor during expiration is different than those of bulk water. The capacitance increase caused by an expiration depends on the width of the pulses used to measure it. The wider the pulses, the more the capacitance increase. In contrast, unwanted capacitance changes such as caused by motion of drops of water on the sensor or by movements of the sensor cable tend to be independent of the width of pulses used to measure capacitance. Thus by measuring the capacitance of the sensor during two unequal pulse intervals and determining the difference in capacitance variations can be minimized.

In the circuit of Fig. 5 the capacitance of the sensor is measured during two unequal pulse intervals and the difference in capacitance during those intervals is determined. Thus, when the electrodes are exposed to hot moist air expired by a patient, water condenses on the electrodes and the capacitance of the sensor increases in dependence on time. If this capacitance is measured during two unequal pulse intervals, the difference in capacitance between the two measurements represents the time dependent portion of the capacitance.

Referring now specifically to Fig. 5, the sensor is generally indicated by reference numeral 10. The battery and associated on/off switch (as battery 34 and switch 36 in Fig. 3) have not been specifically illustrated but the positive side of the battery is connected to the points marked "+V" in the circuit. The circuit is somewhat similar to the circuit of Fig. 3 and incorporates a 7556 dual timing circuit used as two monostable multivibrators $T_2$ and $T_3$ and a series of differential amplifiers denoted $A_1$, $A_2$, etc. An audible alarm is denoted A. The circuit also includes a timer $T_1$, which is a typical 7555 timer.

In operation, timer $T_1$ produces pulses that trigger the multivibrators $T_2$ and $T_3$. The multivibrators in turn produce pulses $P_2$ and $P_3$ respectively the widths of which depend on the mean capacitance of the sensor during those pulses.

A current source for the sensor is provided by amplifier $A_1$ and charges the sensor at a steady rate. When the voltage on the sensor reaches a first trip voltage $V_1$ set by a resistor $R_8$, multivibrator $T_3$ resets, completing pulse $P_3$. The voltage on the sensor continues to rise until it reaches a second trip voltage $V_2$ set by

a second resistor $R_7$. Multivibrator $T_2$ then rests, completing pulse $P_2$ and the sensor is discharged. The respective voltages $V_1$ and $V_2$ are set to different levels by the resistors $R_8$ and $R_7$ so that the pusles $P_2$ and $P_3$ will be unequal.. Thus, as timer $T_1$ runs, respective trains of pulses of unequal length will be produced by the two multivibrators $T_2$ and $T_3$.

The respective trains of pusles from the multivibrators are fed to two amplifiers $A_2$ and $A_3$. The output of amplifier $A_2$ is connected to the negative input side of amplifier $A_3$. The gain of amplifier $A_2$ is set (by resistors $R_{10}$ and $R_{11}$) so that, the input signals to amplifier $A_3$ will be equal under "zero" conditions (sensor exposed to ambient air); there will then be no output signal from amplifier $A_3$.

Under normal operating conditions (sensor exposed to expirations) the output of amplifier $A_2$ represents the mean capacitance during pulses $P_3$ and is fed to amplifier $A_3$, which substracts it from the mean capacitance during pulses $P_2$ represented by the input from multivibrator $T_2$. $A_3$ then provides an integrated output representing the mean capacitance difference of the sensor during the periods of capacitance of the sensor during pulses, $P_3$ is identical to that during corresponding pulses $P_2$, (a condition which would occur during any variations in stray capacitance) amplifier $A_3$ will receive identical inputs and its output will be zero. However, if the mean capacitance is larger

during $P_2$ than during $P_3$ (as during an expiration), the output of $A_3$ will be a positive voltage proportional to the difference in capacitance during the two pulses. Amplifier $A_3$ will then product an output signal. Thus, there will be an output signal from $A_3$ only if the mean capacitance of the sensor during $P_2$ is greater than during pulse $P_3$ (or only if expiration takes place). The respective resistors $R_7$ and $R_8$ will of course be adjusted so that this condition corresponds to proper operation of the sensor.

An output signal from amplifier $A_3$ is AC amplified by amplifier $A_4$ and the peak value of the output of $A_4$ is stored on a capacitor $C_7$. If the next signal peak from $A_4$ is greater than a set percentage of the stored peak (as adjusted a resistor $R_{18}$) then the output of amplifier $A_6$ goes high, charging capacitor $C_8$. Thus, capacitor $C_7$ and resistor $R_{18}$ provide a sensitivity control which adjusts automatically to changes in the sensitivity of the sensor. Capacitor $C_8$ is continuously discharging through resistor $R_{21}$. If a certain period of time elapses without a signal peak, $C_8$ discharges to a level below the voltage set by resistor $R_{20}$ and the output of amplifier $A_7$ goes low, providing an alarm signal.

In this particular embodiment, the alarm A can be connected either to the ouptut of amplifier $A_7$ or the output of amplifier $A_6$ by a single throw double pole

switch denoted $SW_2$. In the position of the switch as shown in Fig. 5, the alarm will sound when an appropriate output signal is received from amplifier $A_7$. If the switch is moved to its other position the alarm will sound each time an output signal is recieved from amplifier $A_6$, i.e. at each expiration. Thus, with the switch in this position, the alarm provides an audible signal representing a patient's breathing rate. Of course, in another embodiment, the alarm could be connected directly to the output of amplifier $A_7$ if the circuit is merely required to provide an alarm signal indicating cessation of breathing.

The circuit also includes a manually operable switch $SW_1$ which is connected in parallel with capacitor $C_7$. The intention is that switch $SW_1$ should be.closed briefly each time the respiration monitor is used and after the patient has taken a few breaths so as to discharge capacitor $C_7$ and effectively "zero" the capacitor so as to avoid the possibility of erroneous signals being derived from a previous peak signal stored on $C_7$.

The circuit of Fig. 5 is also designed to cause operation of the alarm A in an intermittent manner in the event that the voltage of the battery providing power to the circuit falls below a predetermined level. Thus the circuit includes the series combination of a Zener Diod Z and a resistor $R_{24}$ connected in parallel between the respective battery terminals. The junction between Z and $R_{24}$ is connected to the non-inverting input of a differential amplifier $A_8$.  The amplifier output is in turn connected to the alarm A. Since the Zener voltage is constant, the voltage across $R_{24}$ decreases as the battery voltage drops.  When this voltage drops below a trip voltage set by a variable resistor $R_{23}$ the amplifier $A_8$ goes negative sounding the alarm until the positive feedback provided by $C_9$ reverses the output of $A_8$.  This positive feedback causes $A_8$ to oscillate so the alarm will only sound intermittently, conserving power.  The voltage on $R_{23}$ is normally adjusted so that the alarm will sound if the battery voltage drops below 8 volts.

Set out below is a list of the values appropriate to the various components shown in Fig. 5:

Resistors (ohms)

| | |
|---|---|
| $R_1$ = 100K TRIM | $R_{13}$ = 91K |
| $R_2$ = 3M9 | $R_{14}$ = 20K |
| $R_3$ = .56M | $R_{15}$ = .43M |
| $R_4$ = .13M | $R_{16}$ = 2K |
| $R_5$ = .43M | $R_{17}$ = 1K |
| $R_6$ = 1M TRIM | $R_{18}$ = 1M TRIM |
| $R_7$ = .1M TRIM | $R_{19}$ = 4.7M |
| $R_8$ = .1M TRIM | $R_{20}$ = .1M TRIM |
| $R_9$ = .11M | $R_{21}$ = 6.8M |
| $R_{10}$ = .33M | $R_{22}$ = 3.9M |
| $R_{11}$ = 1M | $R_{23}$ = 1M |
| $R_{12}$ = .2M | $R_{24}$ = 1M |

Capacitors (microfarads)

$C_1 = .0039$        $C_6 = .22$

$C_2 = 1$           $C_7 = 68$

$C_3 = 1$           $C_8 = 1.5$

$C_4 = .22$       $C_9 = .33$

$C_5 = 4.7$

$T_1$      = 7555 TIMER

$T_2, T_3$ = 7556 TIMER

$A_1 - A_7$ = 324 QUAD OP.AMP

S = SENSOR

A = ALARM (MOD X20F24C PROJECTS UNLIMITED)

B = 9V BATTERY (MALLORY DURACELL)

It has been found in practical tests that the sensor element provided by the invention is sensitive over its entire length and that a large part of length of the sensor can be disabled by water or saliva without loss of sensing ability; as long as even a small portion of the sensor is exposed to air, the sensor will operate satisfactorily. This is believed to be due to the fact that the conductors of the element make substantially continuous line contact throughout their lengths. This form of sensor element also has the advantage that moisture will tend to travel along the sensor conductors by gravity

18

and accumulate at the sensor housing, reducing the likelihood that the sensor will become disabled due to saturation.

It should of course be noted that, while the preceding description and drawings relate to particular preferred embodiments of the invention, there is no limitation to these particular embodiments. Certain possible modifications have been indicated above and it is to be understood that other changes can also be made.

Within the broad scope of the invention, electrical cricuits other than those specifically described could be used to provide an alarm signal. For example, capacitance changes could be measured in the frequency domain instead of in the time domain as disclosed. The sensor could be connected in a bridge circuit across which an alternating current is applied. In an embodiment designed to minimize the effect of stray capacitance signals (as Fig. 5) the capacitance of the sensor could be measured at two different frequencies and the results compared. For example, two intertwined sensors could be used, each connected in separate bridge circuits oscillating at different frequencies. The outputs from the two circuits could then be combined and their respective outputs compared by a circuit such as Fig. 5 from amplifier $A_3$ on. An alternative would be to use a single sensor with two bridges and respective oscillators together with means for switching the sensor between the two bridges. Alternatively, a single bridge could be alternately excited by two different oscillators.

CLAIMS:

1. A respiration monitor characterized in that it comprises:

a sensor (10;10') comprising a housing (12;12') which defines an air passageway (14;14') and which is adapted to be placed in communication with the airway of a patient so that air inhaled and exhaled by the patient will flow through said passageway (14;14'), and a sensor element (16;16') located in said passageway (14;14') in the path of air flowing therethrough in use, said sensor element comprising a pair of insulated wires (26,28) twisted closely together in at least substantially continuous line contact with one another and forming a capacitor having a dielectric formed partially by the air between said conductors (26,28), whereby changes in the humidity of the air in the passageway (14;14') during inhalation and exhalation by the patient in use will cause corresponding cyclic variations in the capacitance of the said capacitor; and,

electrical circuit means coupled to said conductors (26,28) and adapted to provide an alarm signal in the event of cessation of said cyclic variations in capacitance.

2. A respiration monitor as claimed in claim 1, characterized in that said wires (26,28) are shaped to define a sensor element (16;16') of spiral form.

3. A respiration monitor as claimed in claim 1, characterized in that said housing (12) comprises opposed cylindrical end portions (20,22) which are open at their outer ends and which define respectively opposite ends of said air passageway (14) and an enlarged centre portion (24) receiving said conductors.

4. A respiration monitor as claimed in claim 2, characterized in that said housing (12') comprises first and second generally cylindrical housing portions (42,44) disposed coaxially with respect to one another, said portions having complementary male and female shapes, and wherein the said wires (26,28) are shaped to define a sensor element (16') of generally helical spiral form, said element being disposed in the larger of said housing portions (44) and being located against a shoulder (40) between said portions.

5. A respiration monitor as claimed in claim 1, characterized in that said electrical circuit means comprises: an alarm (A); a timing circuit (32) adapted to normally produce repeated regular output pulses, and to which said sensor (10) is coupled so that changes in the capacitance of said sensor (10) will pulse-width modulate the output of said timing circuit (32); signal processing means adapted to produce a corresponding pulsating amplitude modulated signal from said width modulated pulses; and time delay means adapted to operate said alarm (A) after a predetermined time interval has elapsed following a final pulse from said signal processing means as indicating absence of modulation of said timing circuit (32) pulses as a result of cessation of breathing.

6. A respiration monitor as claimed in claim 1, wherein said electrical circuit means is adapted to measure the capacitance of the sensor under two different circuit conditions, to compare readings obtained from said measurements and to provide an alarm signal in the event that such readings remain the same for a specified length of time.

7. A respiration monitor as claimed in claim 6, wherein said different circuit conditions comprise different frequencies.

8. A respiration monitor as claimed in claim 1, characterized in that said electrical circuit means comprises an alarm (A); timing means $(T_2, T_3)$ adapted to produce two series of repeated pulses $(P_2, P_3)$ of unequal duration and to which the sensor (10') is coupled so that changes in the capacitance of the sensor (10') will pulse-width modulate said pulses; and signal processing means adapted to compare the mean capacitance of the sensor element during the respective series of pulses, said circuit means being adapted to operate the alarm when the mean capacitance of the sensor element remains the same for a predetermined period of time .

9. A respiration monitor as claimed in claim 6, characterized in that said signal processing means is adapted to provide an output signal at each expiration while the mean capacitance of the sensor is different during said respective series of pulses and in that said circuit means further includes switch means adapted to couple said alarm directly to said signal processing means whereby the alarm is operated intermittently each time an output signal is produced by said signal processing means.

10. A sensor for use in respiration monitor, characterized in that it comprises a housing (12;12') which defines an air passageway (14;14') and which is adapted to be placed in communication with the airway of a patient so that air inhaled and exhaled by the patient will flow through said passageway (14;14'), and a sensor element located in said passageway (14;14') in the path of air flowing therethrough in use, said sensor element (16;16') comprising a pair of insulated wires (26,28) twisted closely together in at least substantially continuous line contact with one another and forming a capacitor having a dielectric formed partially by the air between said conductors (26,28), whereby changes in the humidity of the air in said passageway (14;14') during inhalation and exhalation by the patient in use will cause corresponding cyclic variations in the capacitance of the capacitor.

FIG. 1

FIG. 2

FIG. 3

0091522

FIG. 4

FIG. 4a

FIG. 5